# EUROPEAN PATENT SPECIFICATION

(11) **EP 3 120 829 B1**
(45) Date of publication and mention of the grant of the patent: **10.04.2019**
(21) Application number: 15818579.3
(22) Date of filing: 15.05.2015
(51) Int. Cl.: A61K 8/58, A61Q 1/12, A61K 8/29, A61K 8/19, A61Q 1/02, A61K 8/02

(54) **CONTROLLABLE COLOR-CHANGING FOUNDATION CREAM/LIQUID FOUNDATION**
STEUERBARE FARBVERÄNDERNDE FOUNDATIONCREME/FLÜSSIGE FOUNDATION
CRÉME DE FOND DE TEINT/FOND DE TEINT LIQUIDE À CHANGEMENT DE COULEUR CONTRÔLABLE

(30) Priority: 10.07.2014 CN 201410328144
(43) Date of publication of application: 25.01.2017
(73) Proprietor: Shanghai Co-fun Biotech Co., Ltd., Shanghai 201508 (CN)
(72) Inventor: PU, Ke, Shanghai 201508 (CN)
(74) Representative: Ziebig, Marlene
(86) International application number: PCT/CN2015/079027
(87) International publication number: WO 2016/004792

(56) References cited:
- EP-A1- 2 417 960
- WO-A1-2014/051660
- CN-A- 1 602 832
- CN-A- 101 267 798
- CN-A- 102 076 319
- JP-A- H04 338 314
- JP-A- H09 124 319
- JP-A- 2005 002 077
- US-A1- 2010 147 192

## Description

### Field of Invention

The present invention relates to a cosmetic as well as a preparation method thereof, and more particularly to a foundation cream/liquid with a controllable color change, as well as a preparation method thereof.

### Background of the Invention

Foundation cream/liquid is a very important category of cosmetics, which is also a basis for good makeup effects. Cosmetic manufacturers usually provide foundation with different colors (such as dark skin color and light skin color) based on user skin colors. An appearance color and a practical-use color of a common foundation cream are basically the same.

In order to create novel visual effects for attracting consumers, a new type of color-changeable foundation has been developed in recent years. Color change refers to that: during applying, a color of the cream is changed from light (such as pale and white) to dark (such as dark skin color and light skin color); or changed from one (such as light yellow) to another (such as deep red). Such foundation not only covers small wrinkles or blemishes like a common foundation, but also has an obvious color changing effect, which is very novel and suitable for creating a market concept as well as attracting the consumers.

Commercially available color-changeable foundation comprises three types as follows.

### 1) Cellulose adhesion technology

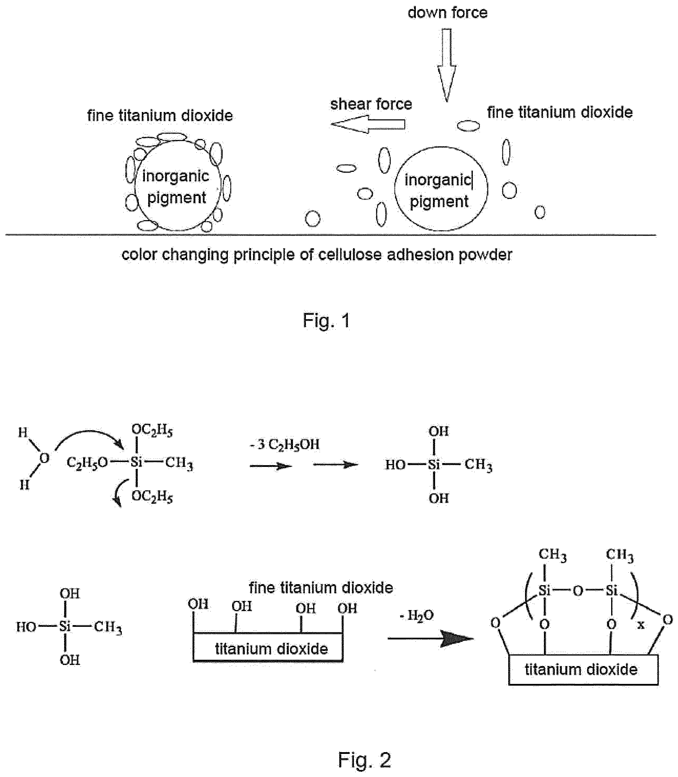

On a surface of inorganic pigments (which usually comprises iron oxide yellow, iron oxide red, iron oxide black, etc.) used in cosmetic foundation, ultra-fine titanium dioxide is adhered by cellulose as an adhesion medium. A preparation method thereof is very close to a Chinese folk traditional food: rice glue ball (which takes fillings as a core, and wet glutinous rice flour is adhered on a surface of the core). In use, after being applied by fingers, the titanium dioxide adhered on the surface of the pigment is separated by a shear force, and an original color of the pigment appears, in such a manner that the foundation changes from white (or pale) to skin color, as shown in fig. 1.

### 2) Color-changeable pearl powder technology

Through refraction and scattering effects of the pearl powder on light, the color changing effect is achieved. When using, the pearl powder, which is emulsified and wrapped in the cream, is applied on skin, in such a manner that the pearl powder is exposed to light for displaying color changing.

### 3) Acid-base indicator

The foundation is prepared with several acid-base indicators according to a principle that when external pH value changes, colors of the acid-base indicators will change. In use, the acid-base indicators contact with the skin (whose pH is slightly acidic), then the color thereof changes, resulting in color changing effect.

In practice, all the above three types of color-changeable foundation have serious defects as follows.

### 1) Defects of the cellulose adhesion technology:

(1) weak covering ability: wherein a particle size (particle diameter) of the inorganic pigment and a shape thereof are generally stable (for example, particle of iron oxide red is generally spherical with a diameter of 0.3-0.5um, and iron oxide black is typically cubic with a diameter of about 0.3um); in order to achieve good adhesion wrapping effect, a particle size of the titanium dioxide should be as small as possible (commonly nano titanium dioxide); furthermore, in order to achieve fast and significant color changing effect, an amount of the titanium dioxide adhered on the surface of the inorganic pigment surface should not be too many, and a thickness of a adhesion layer should not be too thick; therefore, it is difficult to achieve sufficient covering ability;
(2) slow color change:
   during using the foundation, there is a smearing process; iron oxide particles wrapped by the titanium dioxide are broken under the shear force, in such a manner that the iron oxide particles wrapped are exposed for providing the color changing effect;
   therefore, color change speed depends on magnitude of force, speed of smearing, and firmness of adhered particles; wherein slowly and gently smearing is not conducive to breaking the particles, which means not conducive to showing the color changing effect; however, as a cosmetic for face, a foundation cream/liquid is hardly to be rapidly and repeatedly smeared with a larger force during using, which lowers the color changing speed and effect of such foundation;
(3) difficulty of controlling production process:
   firmness cellulose adhesion is a contradiction: if the firmness is too high, the foundation cream/liquid is difficult to provide the color changing effect during using; if the firmness is too low, the particles of the foundation cream/liquid will be broken during producing and packaging, which expose the inorganic pigments too early;
   therefore, during actual production, the cellulose adhered color-changeable foundation requires a lot during production: paddle must not scrape walls, powder need to be pre-soaked following a strict time and temperature, and emulsifying time and temperature must be strictly controlled; otherwise, product quality is difficult to be controlled.

### 2) Defects of the color-changeable pearl powder technology:

(1) insufficient color changing effect:
   before smearing, the color-changeable pearl powder is hidden in the cream and is not exposed to light, so as to hiding color effects of the pearl powder; after smearing, the pearl powder is applied on the skin and exposed to the light, so as to show colors; which is a color changing principle of such technology;
   however, the color effect of the pearl powder is much weaker than that of the iron oxide, while main roles of the foundation cream is to cover blemishes and adjust skin color, where the pearl powder is inadequate;
(2) shiny effect of pearl powder, which is not suitable for entire face:
   the pearl powder naturally has a shiny pearl effect, which is more suitable for is more suitable for eye shadow, lip gloss and other areas in a makeup process; however, the foundation is a make-up basis for the entire face; it will be very unnatural if the entire face is shiny.

### 3) Defects of using acid-base indicators:

(1) there is only a color changing effect without modifying and protecting effects which are essential for foundation products;
   there is only a color changing process, which is not able to cover blemishes and adjust the skin color;
(2) harm to human body without benefit:
   Commonly used acid-base indicators are mainly: nitrophenol, phenolphthalein, thiosulfate phenolphthalein and azo compounds; which basically belong to organic weak acids or organic weak bases; most of these agents are on lists of cosmetic raw materials, which means it is not allowed to use such agents in cosmetics.

### Summary of the Invention

A technical problem to be solved by the present invention is to provide a foundation cream/liquid with a controllable color change and a preparation method thereof. The present invention overcomes defects of the above technologies from a technical origin, wherein a color changing effect of the color-changeable foundation cream/liquid prepared is controllable and rapid; a covering ability is strong; colors are varied; safety and harmlessness are guaranteed; and preparation is simple.

Accordingly, in order to accomplish the above objects, the present invention provides:
pre-mixed powder for a foundation cream/liquid with a controllable color change, comprising: surface treated titanium dioxide obtainable by methyl triethoxy silanization of titanium dioxide and surface treated inorganic pigment obtainable by octyl triethoxy silanization of inorganic pigments, wherein the inorganic pigment is one of iron oxide yellow, iron oxide red, and iron oxide black. According to the present invention, totally different surface treatments are provided to the titanium dioxide and the inorganic pigment (iron oxide) in the foundation cream/liquid, in such a manner that surface properties of such two kinds of powder are quite different. After different surface treatments, the surface properties of the inorganic pigment and the titanium dioxide are quite different as follows.

Octyl groups of eight carbon atoms are connected to an end of a surface silicon atom of the inorganic pigment. Due to being long, a carbochain thereof has a sufficient dispersity in fat of an aliphatic series; while the carbochain has a poor compatibility in oxosilane.

A methyl group of a carbon atom is connected to an end of a surface silicon atom of the titanium dioxide. Due to being short, a carbochain thereof has a sufficient compatibility in the oxosilane.

Therefore, the color-changeable foundation cream/liquid prepared with the pre-mixed powder of the present invention has technical effects such as a controllable and rapid color changing effect, a strong covering ability, varied colors, safety and harmlessness.

According to the pre-mixed powder present invention, the surface treated inorganic pigment comprises iron oxide yellow, iron oxide red and iron oxide black. The inorganic pigment may be other inorganic pigments for foundation. By adjusting contents of the inorganic pigment with different colors, varied foundation cream/liquid is able to be prepared.

According to the pre-mixed powder present invention, detail surface treatment methods may be conventional surface treatment methods as long as same technical effects are guaranteed. After the methyl triethoxy silanization surface treatment, a surface of the titanium dioxide is characterized in that a methyl group of a carbon atom is connected to an end of a surface silicon atom of the titanium dioxide; after the octyl triethoxy silanization surface treatment, a surface of the inorganic pigment is characterized in that octyl groups of eight carbon atoms are connected to an end of a surface silicon atom of the inorganic pigment.

Accordingly, the present invention further provides preferred surface treatment methods, in such a manner that a better surface treatment effect is achieved, the surface properties of the titanium dioxide and the inorganic pigment are more stable, and distribution is evener.

A method for preparing a pre-mixed powder for a foundation cream/liquid with a controllable color change comprises steps of:
thoroughly mixing 0.45kg methyl triethoxy silane with 1.05kg 95% ethanol for obtaining a surface treatment agent just before using;
adding 15kg titanium dioxide into a high-speed mixer; under a high mixing speed, spraying the surface treatment agent prepared into the high-speed mixer with a spray tank, and thoroughly stirring for thoroughly mixing the surface treatment agent with the titanium dioxide; and
discharging powder and instantly sending into an oven, heating at 105°C for 4h, and naturally cooling before discharging.
thoroughly mixing 0.45kg octyl triethoxy silane with 1.05kg 95% ethanol for obtaining a surface treatment agent just before using;
adding 15kg inorganic pigment into a high-speed mixer; under a high mixing speed, spraying the surface treatment agent prepared into the high-speed mixer with a spray tank, and thoroughly stirring for thoroughly mixing the surface treatment agent with the inorganic pigment, wherein the inorganic pigment is one of iron oxide yellow, iron oxide red, and iron oxide black; and
discharging powder and instantly sending into an oven, heating at 105°C, or 70°C if the inorganic pigment is the iron oxide yellow, for 4h, and naturally cooling before discharging.

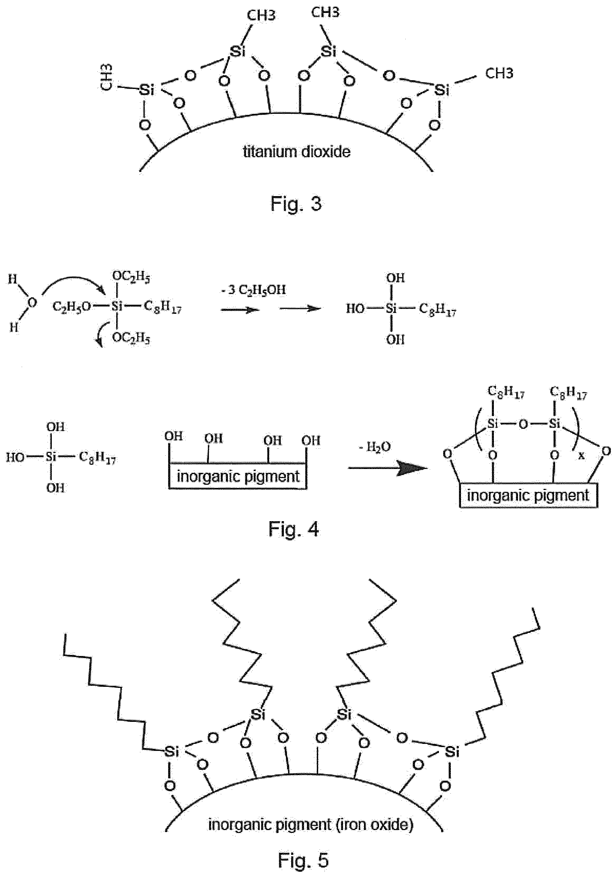

A reaction process is shown in fig. 2, respectively fig. 4. After treatment, surface properties of the powder are shown in fig. 3, respectively fig. 5.

According to the pre-mixed powder present invention, after the surface treatments, a content of the titanium dioxide is preferably 75-95wt%, and a rest is the surface treated inorganic pigment. For example, a preferred color-changeable pre-mixed powder CC807 comprises (wt%):
the titanium dioxide treated by methyl triethoxy silane: 88%;
the iron oxide yellow treated by octyl triethoxy silane: 7.6%;
the iron oxide red treated by the octyl triethoxy silane: 3%;
the iron oxide black treated by the octyl triethoxy silane: 1.4%.

According to the present invention, through certain surface treatment methods, differences between the surface properties of the titanium dioxide and the inorganic pigment are artificially increased; then certain formulas are adopted, in such a manner that the titanium dioxide and the inorganic pigment are in different distribution states in an entire emulsification system, so as to control the color changing effects.

Furthermore, the present invention provides a foundation cream/liquid with a controllable color change, comprising the pre-mixed powder for color-changeable foundation cream/liquid. The foundation cream/liquid further comprises an emulsifier, an emollient, a preservative and an essence according to requirements, the emollient is a silane emollient and so on. A preparation method of the foundation cream/liquid may be a conventional foundation cream/liquid preparation method.

However, for optimizing the color changing effect of the pre-mixed powder of the present invention, technical requirements for emulsifying the pre-mixed powder in the foundation cream/liquid are strict, which means the pre-mixed powder must be fully emulsified in the foundation cream/liquid, and evenly dispersed.

Therefore, according to the present invention, a formula and a preparation method of a color-changeable foundation cream/liquid which specifically use the pre-mixed powder are further optimized, which enables a best effect of the pre-mixed powder during using.

Preferably, a formula and a preparation method of a color-changeable foundation cream/liquid are as follows.

The formula is:

| phase number | group name / provider | content (Wt)% |
|---|---|---|
| A | Abil EM180 (Degussa) | 0.75 |
| | Abil EM90 (Degussa) | 0.75 |
| | Sensil 5060 silane emollient (Shanghai Scinecoo) | 2.20 |
| | DC9040 silicone elastomer (Dow Corning) | 1.00 |
| | DC345 (Dow Corning) | 5.00 |
| | DC556 (Dow Corning) | 1.60 |
| | DC200 (5CST) (Dow Corning) | 5.00 |
| B | color-changeable pre-mixed powder | 8.00 |
| C | deionized water | 100 |
| | 1% ethyloic-β-glucan (Angel Yeast C90) | 3.00 |
| | sodium chloride | 1.00 |
| | betaine(trimethylglycine) | 3.00 |
| | 1,3-butanediol | 15.00 |
| D | preservative | appropriate amount |
| | essence | appropriate amount |

The preparation method comprises steps of:
   1) mixing and stirring an A phase, keeping homogeneity for lmin, and thoroughly dispersing with a DC9040 silicone elastomer;
   2) heating the A phase to 55-60°C, then adding B phase powder, and keeping homogeneity for lmin for thoroughly dispersing the powder;
   3) meanwhile, heating a C phase to about 55-60°C for completely melting;
   4) increasing a stirring speed of the A and B phases (to 200-300rpm), slowly adding the C phase into the A and B phases for completely emulsifying;
   5) cooling by stirring until a temperature is lower than 45°C, then adding a D phase in such a manner that cream becomes white, and discharging; and
   6) during cooling, keeping a stirring speed (at 200-300rpm), wherein the cream becomes white within 2-3h.

With the foregoing formula and preparation method, a certain formula and an optimized method are adopted, wherein with a specific technique of mixing the A, B, C and D phases, the pre-mixed powder of the present invention is fully emulsified in the foundation cream/liquid, and is evenly dispersed. Furthermore, color is rapidly changed during using, which optimizes an effect.

Accordingly, advantages of the present invention are as follows.

### 1) Rapid color change

After gently applying the color-changeable foundation of the present invention on skin, the color changing effect will instantly show.

### 2) Strong covering ability

According to the color-changeable foundation of the present invention, a particle size thereof is not limited, and an addition amount in the formula is not limited much, while an excellent covering ability is achieved.

### 3) Varied colors and adjustable chroma

According to the color-changeable foundation of the present invention, an addition amount of the titanium dioxide in the formula is not limited much, which enables a very good coloring effect.

### 4) Safety

The color-changeable foundation treated by the present invention comprises no acid-base indicator, thereby causing no harm to the skin. The above surface treatment methods have been practiced for many years in cosmetic applications, which are proved to be safe and harmless.

### 5) Simple production technique

The production technique is simple and controllable, and materials used are shear-resistant, extrusion-resistant, and vibration-resistance, which are suitable for transportation by cosmetic manufacturers.

In order to achieve a sufficient color effect, the color-changeable foundation formula designed by the present invention adopts the emulsion system with silicone oil wrapping water. Although the foundation feels silky, breathable and comfortable, a moisture effect thereof is insufficient. Therefore, in autumns and winters, other skin care products are needed.

### Brief Description of the Drawings

Fig. 1 illustrates a principle of a cellulose adhesion technology according to a prior art.
Fig. 2 illustrates a reaction process of a surface treatment of titanium dioxide according to the present invention.
Fig. 3 illustrates a surface property of powder of the titanium dioxide after the surface treatment according to the present invention.
Fig. 4 illustrates a reaction process of a surface treatment of an inorganic pigment according to the present invention.
Fig. 5 illustrates a surface property of powder of the inorganic pigment after the surface treatment according to the present invention.

### Embodiments

Referring to preferred embodiments, the present invention is further illustrated.

### Preferred embodiment 1:

A method of a methyl triethoxy silanization surface treatment of titanium dioxide comprises steps of:
thoroughly mixing 0.45kg methyl triethoxy silane with 1.05kg 95% ethanol for obtaining a surface treatment agent just before using;
adding 15kg titanium dioxide into a high-speed mixer; under a high mixing speed, spraying the surface treatment agent prepared into the high-speed mixer with a spray tank, and thoroughly stirring for thoroughly mixing the surface treatment agent with the titanium dioxide; and
discharging powder and instantly sending into an oven, heating at 105°C for 4h, and naturally cooling before discharging.

A method of the octyl triethoxy silanization surface treatment preferably comprises steps of:
thoroughly mixing 0.45kg octyl triethoxy silane with 1.05kg 95% ethanol for obtaining a surface treatment agent just before using;
adding 15kg inorganic pigment into a high-speed mixer; under a high mixing speed, spraying the surface treatment agent prepared into the high-speed mixer with a spray tank, and thoroughly stirring for thoroughly mixing the surface treatment agent with the inorganic pigment; and
discharging powder and instantly sending into an oven, heating at 105°C, or 70°C if the inorganic pigment is the iron oxide yellow, for 4h, and naturally cooling before discharging.

Preferred embodiment 2: color-changeable pre-mixed powder with different proportions, wherein following proportions are all based on mass fraction.

| component name | CC803 content (%) | CC805 content (%) | CC807 content (%) | CC808 content (%) |
|---|---|---|---|---|
| titanium dioxide treated by methyl triethxy silane | 88 | 90 | 85 | 91 |
| iron oxide yellow treated by octyl triethxy silane | 8.7 | 8 | 9.6 | 5.4 |
| iron oxide red treated by octyl triethxy silane | 2.3 | 1.4 | 4 | 2.3 |
| iron oxide black treated by octyl triethxy silane | 1 | 0.6 | 1.4 | 1.3 |

Adjustment of the above proportions only affects the color of the foundation products, wherein the proportions are free to change according to a desired color of the foundation products. As a basis of make-up, the content of the titanium dioxide is usually at 75%-95%, wherein a higher content looks too white, while a lower content looks too dark, which are not conducive to a sufficient make-up effect.

### Preferred embodiment 3: color-changeable foundation cream:

Formulas are as follows.

| phase number | group name / provider | content (Wt)% | | |
|---|---|---|---|---|
| | | formula1 | formula2 | formula3 |
| A | Abil EM180 (Degussa) | 0.75 | 0.75 | 0.70 |
| | Abil EM90 (Degussa) | 0.75 | 0.75 | 0.70 |
| | Sensil 5060 silane emollient (Shanghai Scinecoo) | 2.20 | 2.20 | 2.20 |
| | DC9040 silicone elastomer (Dow Corning) | 1.00 | 1.00 | 1.00 |
| | DC345 (Dow Corning) | 5.00 | 5.00 | 6.00 |
| | DC556 (Dow Corning) | 1.60 | 1.60 | 1.60 |
| | DC200 (5CST) (Dow Corning) | 5.00 | 5.00 | 6.00 |
| B | color-changeable pre-mixed powder CC807/CC803/CC805/CC808 | 8.00 | 10.0 | 11.0 |
| C | deionized water | 100 | 100 | 100 |
| | 1% ethyloic-β-glucan (Angel Yeast C90) | 3.00 | 3.00 | 3.00 |
| | sodium chloride | 1.00 | 1.00 | 1.00 |
| | betaine(trimethylglycine) | 3.00 | 3.00 | 3.00 |
| | 1,3-butanediol | 15.00 | 15.00 | 15.00 |
| D | preservative | appropriate amount | appropriate amount | appropriate amount |
| | essence | appropriate amount | appropriate amount | appropriate amount |

A preparation method comprises steps of:
1) mixing and stirring an A phase, keeping homogeneity for lmin, and thoroughly dispersing with a DC9040 silicone elastomer;
2) heating the A phase to 55-60°C, then adding B phase powder, and keeping homogeneity for lmin for thoroughly dispersing the powder;
3) meanwhile, heating a C phase to about 55-60°C for completely melting;
4) increasing a stirring speed of the A and B phases (to 200-300rpm), slowly adding the C phase into the A and B phases for completely emulsifying;
5) cooling by stirring until a temperature is lower than 45°C, then adding a D phase in such a manner that cream becomes white, and discharging; and
6) during cooling, keeping a stirring speed (at 200-300rpm), wherein the cream becomes white within 2-3h.

In practice, the foundation product according to the preferred embodiment 3 is significant in the color changing effect, rapid in color change, strong in covering ability, easy to evenly smearing, breathable and easy to be absorbed. 30 testers were invited for trying the present invention (all females, 18-45 years old; testing time: May. 2014; testing positions: 15 testers in Shanghai and 15 testers in Guangzhou), wherein feedbacks (multiple-choice) thereof were collected as follows.

| | |
|---|---|
| super fast color change, which is white at the beginning, and instantly turns to skin color or nude color, it is amazing, very interesting | 28 chose |
| good covering ability after color change | 22 chose |
| soft and silky | 18 chose |
| very breathable | 14 chose |
| sufficient endurance | 9 chose |
| slightly dry | 6 chose |
| willing to buy when commercially available | 27 chose |

### Industrial practicability

According to the present invention, the formula of the foundation cream/liquid with the controllable color change and the preparation method thereof adopt a certain formula and an optimized method, wherein with a specific technique of mixing the A, B, C and D phases, the pre-mixed powder of the present invention is fully emulsified in the foundation cream/liquid, and is evenly dispersed. Furthermore, color is rapidly changed during using, which optimizes an effect. According to the present invention, the foundation cream/liquid with the controllable color change has a rapid color changing effect, a strong covering ability, varied colors, adjustable chroma, safety, and simple production processes, which enables a great market prospect and a strong industrial practicability.

## Claims

1. Pre-mixed powder for a foundation cream/liquid with a controllable color change, comprising: surface treated titanium dioxide obtainable by methyl triethoxy silanization of titanium dioxide and a surface treated inorganic pigment obtainable by octyl triethoxy silanization of inorganic pigments, wherein the inorganic pigment is one of iron oxide yellow, iron oxide red and iron oxide black.

2. The pre-mixed powder of claim 1, wherein a content of the surface treated titanium dioxide is 75-95wt% and a rest of the pre-mixed powder is surface treated inorganic pigment.

3. A method for preparing a pre-mixed powder for a foundation cream/liquid with a controllable color change, the method comprising the steps of:
thoroughly mixing 0.45kg methyl triethoxy silane with 1.05kg 95% ethanol for obtaining a surface treatment agent just before using;
adding 15kg titanium dioxide into a high-speed mixer; under a high mixing speed, spraying the surface treatment agent prepared into the high-speed mixer with a spray tank, and thoroughly stirring for thoroughly mixing the surface treatment agent with the titanium dioxide; and
discharging powder and instantly sending into an oven, heating at 105°C for 4h, and naturally cooling before discharging;
thoroughly mixing 0.45kg octyl triethoxy silane with 1.05kg 95% ethanol for obtaining a surface treatment agent just before using;
adding 15kg inorganic pigment into a high-speed mixer; under a high mixing speed, spraying the surface treatment agent prepared into the high-speed mixer with a spray tank, and thoroughly stirring for thoroughly mixing the surface treatment agent with the inorganic pigment, wherein the inorganic pigment is one of iron oxide yellow, iron oxide red and iron oxide black; and
discharging powder and instantly sending into an oven, heating at 105°C, or 70°C if the inorganic pigment is the iron oxide yellow, for 4h, and naturally cooling before discharging.

4. The method of claim 3, wherein after the methyl triethoxy silanization surface treatment and the octyl triethoxy silanization surface treatment, a content of the surface treated titanium dioxide is 75-95wt%, and a rest is the surface treated inorganic pigment.

5. A foundation cream/liquid with a controllable color change, comprising pre-mixed powder for the foundation cream/liquid with the controllable color change, as recited in claim 1 or 2.

6. The foundation cream/liquid, as recited in claim 5, further comprising an emulsifier, an emollient, a preservative and an essence.

7. The foundation cream/liquid, as recited in claim 6, wherein the emollient is a silane emollient.

8. The foundation cream/liquid, as recited in claim 5, wherein the pre-mixed powder is fully emulsified in the foundation cream/liquid, and is evenly dispersed.

## Patentansprüche

1. Vorgemischtes Puder für eine Foundationcreme/flüssige Foundation mit steuerbarer Farbveränderung, umfassend: oberflächenbehandeltes Titandioxid, hergestellt durch Methyltriethoxysilanisierung von Titandioxid, und ein oberflächenbehandeltes anorganisches Pigment, hergestellt durch Octyltriethoxysilanisierung anorganischer Pigmente, wobei das anorganische Pigment eines aus gelbem Eisenoxid, rotem Eisenoxid und schwarzem Eisenoxid ist.

2. Vorgemischtes Puder nach Anspruch 1, wobei ein Gehalt des oberflächenbehandelten Titaniumdioxids 75-95 Gew-% beträgt und der Rest des vorgemischten Puders oberflächenbehandeltes anorganisches Pigment ist.

3. Verfahren zur Herstellung eines vorgemischten Puders für eine Foundationcreme/flüssige Foundation mit steuerbarer Farbveränderung, wobei das Verfahren folgende Schritte umfasst:
gründliches Mischen von 0,45 kg Methyltriethoxysilan mit 1,05 kg 95 %igem Ethanol zur Gewinnung eines Oberflächenbehandlungsmittels direkt vor der Verwendung;
Hinzufügen von 15 kg Titandioxid in einen Hochgeschwindigkeitsmischer; unter hoher Mischgeschwindigkeit, Sprühen des hergestellten Oberflächenbehandlungsmittels in den Hochgeschwindgkeitsmischer mithilfe eines Sprühtanks, und gründliches Rühren zum gründlichen Mischen des Oberflächenbehandlungsmittels mit dem Titandioxid; und
Entladen des Puders und unmittelbare Übertragung in einen Ofen, Erhitzen auf 105 °C für 4 Stunden, und natürliches Abkühlen vor dem Entladen;
gründliches Mischen von 0,45 kg Octyltriethoxysilan mit 1,05 kg 95%igem Ethanol zur Gewinnung eines Oberflächenbehandlungsmittels direkt vor der Verwendung;
Hinzufügen von 15 kg anorganischem Pigment in einen Hochgeschwindigkeitsmischer; unter hoher Mischgeschwindigkeit, Sprühen des hergestellten Oberflächenbehandlungsmittels in den Hochgeschwindigkeitsmischer mithilfe eines Sprühtanks, und
gründliches Rühren zum gründlichen Mischen des Oberflächenbehandlungsmittels mit dem anorganischen Pigment, wobei das anorganische Pigment eines aus gelbem Eisenoxid, rotem Eisenoxid und schwarzem Eisenoxid ist; und
Entladen des Puders und unmittelbare Übertragung in einen Ofen, Erhitzen auf 105 °C, oder 70 °C wenn das anorganische Pigment gelbes Eisenoxid ist, für 4 Stunden, und natürliches Abkühlen vor dem Entladen;

4. Verfahren nach Anspruch 3, wobei nach der Methyltriethoxysilanisierungs-Oberflächenbehandlung und der Octyltriethoxysilanisierungs-Oberflächenbehandlung ein Gehalt des oberflächenbehandelten Titandioxid 75-95 Gew-% beträgt und der Rest das oberflächenbehandelte anorganische Pigment ist.

5. Foundationcreme/flüssige Foundation mit einer steuerbaren Farbveränderung, umfassend vorgemischtes Puder für die Foundationcreme/flüssige Foundation mit der steuerbaren Farbveränderung nach Anspruch 1 oder 2.

6. Foundationcreme/flüssige Foundation nach Anspruch 5, ferner umfassend einen Emulgator, einen Weichmacher, ein Konservierungsmittel und eine Essenz.

7. Foundationcreme/flüssige Foundation nach Anspruch 6, wobei der Weichmacher ein Silan-Weichmacher ist.

8. Foundationcreme/flüssige Foundation nach Anspruch 5, wobei das vorgemischte Puder vollständig in der Foundationcreme/flüssigen Foundation emulgiert und gleischmäßig dispergiert ist.

## Revendications

1. Poudre prémélangée pour une crème de fond de teint/un fond de teint liquide avec changement de couleur contrôlable, comprenant : un dioxyde de titane traité en surface obtenu par silanisation triéthoxyméthyl de dioxyde de titane, et un pigment inorganique traité en surface obtenu par silanisation triéthoxy-octyl de pigments inorganiques, le pigment inorganique étant un pigment sélectionné parmi l'oxyde de fer jaune, l'oxyde de fer rouge et l'oxyde de fer noir.

2. Poudre prémélangée selon la revendication 1, un contenu du dioxyde de titane traité en surface étant de 75-95 % massique et un reste de la poudre prémélangée étant un pigment inorganique traité en surface.

3. Procédé pour préparer une poudre prémélangée pour une crème de fond de teint/un fond de teint liquide avec changement de couleur contrôlable, le procédé comprenant les étapes de :
mélanger soigneusement 0,45 kg de triéthoxyméthylsilane et 1,05 kg d'éthanol à 95 % afin d'obtenir un agent de traitement de surface juste avant utilisation ;
ajouter 15 kg de dioxyde de titane dans un mélangeur à grande vitesse ; à grande vitesse de mélange, pulvériser l'agent de traitement de surface préparé dans le mélangeur à grande vitesse avec un réservoir de pulvérisation, et agiter soigneusement pour mélanger soigneusement l'agent de traitement de surface et le dioxyde de titane ; et
déverser la poudre et immédiatement mettre dans un four, chauffer à 105 °C pendant 4 heures, et laisser refroidir naturellement avant déversement ;
mélanger soigneusement 0,45 kg de triéthoxy-octyl-silane et 1,05 kg d'éthanol à 95 % afin d'obtenir un agent de traitement de surface juste avant utilisation ;
ajouter 15 kg de pigment inorganique dans un mélangeur à grande vitesse ; à grande vitesse de mélange, pulvériser l'agent de traitement de surface préparé dans le mélangeur à grande vitesse avec un réservoir de pulvérisation, et
agiter soigneusement pour mélanger soigneusement l'agent de traitement de surface et le pigment inorganique, le pigment inorganique étant un pigment parmi l'oxyde de fer jaune, l'oxyde de fer rouge et l'oxyde de fer noir ; et
déverser la poudre et immédiatement mettre dans un four, chauffer à 105 °C, ou à 70 °C si le pigment inorganique est l'oxyde de fer jaune, pendant 4 heures, et laisser refroidir naturellement avant déversement.

4. Procédé selon la revendication 3, une teneur du dioxyde de titane traité en surface étant de 75-95 % massique et un reste étant le pigment inorganique traité en surface, après le traitement de surface par silanisation triéthoxyméthyl et le traitement de surface par silanisation triéthoxy-octyl.

5. Crème de fond de teint/fond de teint liquide avec changement de couleur contrôlable, comprenant la poudre prémélangée pour la crème de fond de teint/le fond de teint liquide avec le changement de couleur contrôlable selon la revendication 1 ou 2.

6. Crème de fond de teint/fond de teint liquide selon la revendication 5, comprenant en outre un émulsifiant, un émollient, un agent de conservation et une essence.

7. Crème de fond de teint/fond de teint liquide selon la revendication 6, l'émollient étant un émollient de silane.

8. Crème de fond de teint/fond de teint liquide selon la revendication 5, la poudre prémélangée étant totalement émulsionnée dans la crème de fond de teint/dans le fond de teint liquide et étant dispersée de façon homogène.
